# EUROPÄISCHE PATENTSCHRIFT

(11) **EP 1 079 838 B1**
(45) Veröffentlichungstag und Bekanntmachung des Hinweises auf die Patenterteilung: **11.09.2002**
(21) Anmeldenummer: 99952070.3
(22) Anmeldetag: 11.05.1999
(51) Int. Cl.: A61K 31/66

(54) **EDELFOSIN ZUR BEHANDLUNG VON HIRNTUMOREN**
EDELFOSIN FOR THE TREATMENT OF BRAIN TUMOURS
EDELFOSINE POUR LE TRAITEMENT DE TUMEURS DU CERVEAU

(30) Priorität: 19.05.1998 DE 19822509
(43) Veröffentlichungstag der Anmeldung: 07.03.2001
(73) Patentinhaber: MEDMARK PHARMA GMBH, 82041 Oberhaching (DE)
(72) Erfinder: NAGLER, Apollonia, D-82031 Grünwald (DE)
(74) Vertreter: Huber, Bernhard, Dipl.-Chem.
(86) Internationale Anmeldenummer: EP9903241
(87) Internationale Veröffentlichungsnummer: WO99059599

(56) Entgegenhaltungen:
- ZELLER W J ET AL: "Interstitial chemotherapy of experimental gliomas." CANCER TREATMENT REVIEWS, (1990 SEP) 17 (2-3) 183-9. , XP002116962
- ENGEBRAATEN, OLAV ET AL: "Effect of alkyl-lysophospholipid on glioblastoma cell invasion into fetal rat brain tissue in vitro" CANCER RES. (1991), 51(6), 1713-19 , XP002116963
- BERDEL, WOLFGANG E. ET AL: "Cytotoxicity of alkyl-lysophospholipid derivatives and low-alkyl-cleavage enzyme activities in rat brain tumor cells" CANCER RES. (1983), 43(2), 541-5 , XP002116964
- BERDEL, WOLFGANG E. ET AL: "Cytotoxic effects of alkyl-lysophospholipids in human brain tumor cells" ONCOLOGY (1984), 41(2), 140-5 , XP002116965
- UNGER, CLEMENS ET AL: "Blood- brain barrier and penetration of cytostatics" KLIN. WOCHENSCHR. (1985), 63(12), 565-71 , XP002116966

## Beschreibung

Die Erfindung betrifft Edelfosin (INN; 1-Octadecyl-2-methyl-sn-glycero-3-phosphocholin, häufig auch als ET180CH3 bezeichnet) zur Herstellung eines Arzneimittels zur Behandlung von primären und sekundären Hirntumoren ausgehend von soliden und nicht-soliden Tumoren.

Die Dauerheilung eines malignen Hirntumors ist nach heutigem Wissensstand nicht möglich, d.h. es gibt für maligne Gliome derzeit keine kurativen Therapieansätze. Grundsätzlich wird eine individuelle Behandlung unter Berücksichtigung evtl. Funktionseinbußen durch operative Maßnahmen angestrebt und eine Verlängerung der Überlebenszeit bei bestmöglicher Lebensqualität.

Die Inzidenz von Hirntumoren ist nach wie vor steigend und umso ausgeprägter, je älter die Menschen sind. So erkranken z.B. durchschnittlich 1.8/ 100.000 Personen im Alter von 15 - 24 Jahren, aber rd. 18.4/100.000 im Alter von 65 - 79 Jahren. Der Alterspeak liegt zwischen 55 und 73 Jahren, wobei in den letzten Jahren zunehmend mehr jugendliche Patienten mit Glioblastomen erfasst werden. In Deutschland erkranken jährlich ca. 7.000 Menschen an einem primären Hirntumor, von denen der größte Teil innerhalb des 1. Jahres verstirbt. Trotz Operation und Bestrahlung leben die Menschen im Durchschnitt nur noch elf Monate. Dann hat sich der Tumor so stark vergrößert und sind so große Mengen Flüssigkeiten ausgetreten, dass der Hirnstamm gequetscht wird und lebenswichtige Funktionen aussetzen.

Die am häufigsten auftretenden Tumoren nehmen ihren Ausgang von Astrozyten, Ependymozyten sowie Oligodendrozyten. Die Prognose von Hirntumoren ist prinzipiell schlecht. Maligne Gliome sind die am weitesten verbreiteten Hirntumore, von denen wiederum das Glioblastoma multiforme und das anaplastische Astrozytom, die zusammen rd. 80% aller malignen Gliome ausmachen, die schlechteste Prognose haben. Ausgerechnet bei diesen Tumoren ist häufig nur eine Teilresektion möglich.

Die derzeit verfügbaren nicht-operativen Therapiemöglichkeiten - Bestrahlung und Chemotherapie - sind ausnahmslos mit z.T. schweren unerwünschten Arzneimittelwirkungen verbunden, was besonders für chemotherapeutische Therapieansätze bei einer immer älter werdenden Bevölkerung mit Mehrfacherkrankungen einen limitierenden Faktor darstellt.

Eines der Hauptprobleme aber ist die Tatsache, dass auch oder trotz Anwendung von aggressiven Therapieschemata die Rezidivierung dieser Tumore unvermeidbar ist. Eine Dauertherapie mit Chemotherapeutika ist aufgrund der Toxizität dieser Substanzen nicht möglich, eine wiederholte Anwendung nicht sinnvoll, da die Ansprechraten dann gegen null tendieren. Die Grenze der Strahlenbelastung ist ebenso relativ schnell erreicht, will man bereits den Primärtumor effektiv behandeln. So sieht man sich auch heute noch einer Situation gegenüber, in der nach Abschluss der ersten Therapieregime "abgewartet" werden muss, bis ein erneutes Rezidiv entstanden ist. Daran kann auch eine noch so engmaschige Kontrolle nichts ändern.

Prinzipiell werden primäre Hirntumore als Tumoren eingestuft, für die es auch heute noch keine kurativen, effektiven Therapieansätze gibt. in Italien wurden in einer jüngsten Entscheidung die malignen Gliome gar als "orphan drug"-Indikation eingestuft, weil wirksame Therapieansätze fehlen. Dazu kommt, dass von jährlich weltweit ca. 2.000.000 berichteten Krebserkrankungen rund 800.000 - das sind 40% - à priori chemotherapieresistent sind, echte Therapiealternativen somit auch heute noch dringender denn je notwendig sind.

### Derzeit verwendete Therapieansätze

Maligne Hirntumore zählen zu den bösartigsten Tumoren überhaupt; ihre Prognose ist infaust. Selbst mit einer Kombination von Operation, Bestrahlung und Chemotherapie beträgt die Überlebenszeit ab Zeitpunkt Diagnose bei Patienten mit hochgradig differenzierten Gliomen i.d.R. weniger als 1 Jahr.

Die operative Entfernung eines malignen Tumors mit totaler Tumorentfernung, d.h. mit kurativer Zielsetzung ist stets die Therapie der Wahl. Allerdings liegt oft Inoperabilität vor; so sind z.B. von allen diagnostizierten Astrozytomen nur etwas mehr als 20% operabel, in den allermeisten Fällen ist nur eine Teilresektion möglich.

Die einzige Möglichkeit bleibt daher oftmals die radioaktive Bestrahlung, die allerdings ebenfalls mit Nebenwirkungen verbunden ist und den Nachteil einer nur lokal anwendbaren Therapie hat. Die meisten Tumorherde haben einen unscharf begrenzten Rand, der auch mit Computertomographie (CT) oder Magnet-Resonanz-Therapie (MRT) nicht darstellbar ist, so dass zwangsweise ein beträchtlicher Anteil gesunden Gewebes in das zu bestrahlende Areal einbezogen werden muss. Die Toleranz oder fehlende Widerstandsfähigkeit dieses gesunden Gewebes ist daher der limitierende Faktor. Da aber die lokalen Behandlungsmodalitäten immer aggressiver werden, ist von einer Zunahme der strahlungsbedingten Schäden auszugehen. Solche sind entweder 1. akute Reaktionen: Entwicklung eines Ödems innerhalb weniger Stunden, begleitet von Kopfschmerzen, Übelkeit/Erbrechen, Somnolenz, Fieber, Verschlechterung neurologischer Symptome; 2. frühe-verzögerte Reaktionen: wenige Wochen bis 4 Monate nach Bestrahlung aufgrund vorübergehender Entmyelinisierung oder strahlungsbedingter Änderung der Permeabilität der Kapillaren; äußert sich in vorübergehender neurologischer Verschlechterung, Somnolenz sowie fokaler (fokal = von einem Herd ausgehend) Enzephalopathie, 3. späte Reaktionen: mehrere Monate bis Jahre nach der Behandlung. Diese sind eigentlich die schwersten und äußern sich in Schlaganfällen, neuropsychologischen Störungen, Demenz, Atrophie der cerebralen Cortex (bei bis zu 39% der Patienten mit Ganzhirnbestrahlung).

Chemotherapien werden prinzipiell nur in Form einer adjuvanten Therapie angewendet, d.h. unmittelbar nach chirurgischer Entfernung des Tumors. Allerdings versagt bei Hirntumoren die Chemotherapie fast immer, weil die Blut-Hirn-Schranke - eine natürliche Barriere gegen Giftstoffe und Krankheitserreger - das Eindringen der meisten Arzneimittel ins Gehirn verhindert. Es gibt zwar einige Medikamente (überwiegend aus der Substanzklasse der Nitrosoharnstoffe), die die Blut-Hirn-Schranke zu überwinden vermögen, allerdings ist ihr Effekt höchst umstritten. Entsprechend ist es bis heute nicht gelungen, eine Chemo-Standardtherapie zu etablieren. Die WHO klassifiziert entsprechend Hirntumore als Tumoren, die nicht oder nur äußerst marginal sensitiv gegenüber einer zytotoxischen Behandlung sind.

Selbst die neuesten entwickelten Arzneimittel bedeuten keinen echten Fortschritt im Sinne neuer molekularer Therapieansätze. Das Zytostatikum Carmustin, ein Nitrosoharnstoffderivat, wird in Form medikamentengetränkter Plättchen bereits intraoperativ in die Tumorhöhle eingepflanzt, zeigt aber selbst dann nur einen moderaten bis fehlenden Effekt. Abgesehen davon, ist es nur für die operablen Fälle anwendbar. Ferner hat dieses Zytostatikum, wird es systemisch gegeben, wie alle bekannten Zytostatika als nicht selektiv wirkendes Molekül einen schädigenden Einfluss auf die Normalzellen des Körpers, d.h. seine Anwendung geht mit entsprechenden unerwünschten Nebenwirkungen einher (wie z.B. Erbgutschädigung, pulmonale Toxizität, Myelosuppression u.a.). Dies trifft auch für andere, ab und zu für die Behandlung von Hirntumoren verwendeten Zytostatika wie "Procarbazin", "Cyclophosphamid", "Vincristin" zu. In vielen Behandlungszentren werden daher Patienten mit Glioblastomen und schlechten prognostischen Kriterien von vorneherein von einer Chemotherapie ausgeschlossen.

Aufgrund der prinzipiell fehlenden oder allenfalls moderaten Wirksamkeit der bekannten Zytostatika bei primären oder sekundären Hirntumoren kommt letztlich der Frage, ob zytotoxische Substanzen die Blut-Hirn-Schranke zu überwinden vermögen oder nicht, rein akademischer Charakter zu (vgl. DeVita & al. 1997, S. 2041).

Aufgabe der Erfindung ist es daher, ein Arzneimittel zur Behandlung von Hirntumoren zu schaffen, welches die folgenden Vorteile aufweist:
a) bessere Schrankengängigkeit des Medikamentes
b) reproduzierbare pharmakodynamische Wirkung
c) therapeutische Wirksamkeit auch in der *ultima ratio*-Situation
d) keine Organtoxizität
e) Gewährleistung eines Maximums an Lebensqualität bei akzeptablen unerwünschten Arzneimittelwirkungen (UAW)
f) vereinfachte Anwendung, möglichst ambulante Therapie
g) uneingeschränkte Therapiedauer
Gelöst wird diese Aufgabe erfindungsgemäß durch die Verwendung von 1-Octadecyl-2-methyl-sn-glycero-3-phosphocholin (Edelfosin) zur Herstellung eines Arzneimittels zur oralen Verabreichung zur Behandlung von Hirntumoren. Edelfosin kann dabei in dcer L-Form, der D-Form oder als Razemat eingesetzt werden.

Edelfosin hat zwei Vorteile, die seine Verwendbarkeit bei der Therapie von primären (d.h. von Hirnzellen ausgehenden) und sekundären (also von nicht hirneigenen Körperzellen ausgehenden, aber im Hirn auftretenden) Hirntumoren als bestens geeignet erscheinen fassen.

Zum einen wirkt das Molekül strikt **selektiv** (vgl. Hickman 1992, Fig. 7). Nach Aufnahme in die Zellen entfaltet es seine zytostatische Wirkung nur auf die entarteten Zellen, während es in gesunden Zellen abgebaut wird (vgl. Magistrelli & al. 1994, Tab. 2). Diese Selektivität bedeutet zum einen, dass das Molekül weder mutagen (vgl. King & al. 1981), noch kanzerogen (vgl. Berdel & al. 1983; Berger & al. 1984), noch teratogen/ embryotoxisch oder chromosenschädigend ist (vgl. Bauchinger & al. 1983). Die selektive Wirkung von Edelfosin hat auch zur Folge, dass sich die durch das Medikament verursachten unerwünschten Arzneimittelwirkungen ganz deutlich in Schwere, Grad und Dauer von der der bekannten zytotoxischen Chemotherapeutika unterscheiden. Aufgrund dieser Eigenheit kann die Therapie mit Edelfosin problemlos ambulant durchgeführt werden.

Ein vor der Erfindung nicht gelöstes Problem bei der Behandlung von Krebs besteht in der begrenzten **Dauer der Anwendung** einer Therapie. Die Toxizität der heute verfügbaren Medikamente erlaubt zum einen eine Anwendung nur in Zyklen und zum anderen nur über eine begrenzte Zeit. Tumorzellen, denen die Information für den genetisch programmierten Zelltod (Apoptose) "verloren" gegangen ist, sind aber *de facto* unsterblich. Dazu kommt, dass die heute verwendeten medikamentösen Therapien entweder einen bestimmten Rezeptorstatus oder aber ein bestimmtes Stadium des Zellzyklus voraussetzen. Somit können während der ohnehin zeitlich begrenzten Therapiedauer nicht alle Zellen, sondern nur ein Teil davon effektiv angegangen werden.

Diese Probleme sind mit der Erfindung gelöst. Zum einen entfaltet das Molekül seine **Wirkung nicht über Rezeptoren** (vgl. Snyder & al. 1991); zum anderen ist seine Wirkung nicht vom Teilungsstadium einer Zelle abhängig, sondern wirkt über Enzyme, die in jeder Phase eines Zellzyklus aktiv sind und essentiell vorhanden sein müssen.

Aufgrund der nachgewiesenen Selektivität von Edelfosin kann das Medikament über einen praktisch unbegrenzten Zeitraum als Dauertherapie gegeben werden, so dass die Hirntumorzellen dauerhaft in ihrem Teilungsverhalten beeinflusst werden. Die längste bisher bekannte Therapiedauer bei einem Hinrtumorpatienten reicht inzwischen über 6 Jahre. Bei einer Gesamtdosis von ca. 640 g war der Patient bis zu seiner Pensionierung berufstätig und litt unter keinen nennenswerten unerwünschten Arzneimittelwirkungen (vgl. Tabelle 1, Spalte "Pat." - dort Nr. 542). Edelfosin läßt sich leicht oral verabreichen, zweckmäßig in einem trinkbaren Trägermittel gelöst. Vorzugsweise werden Trägermittel auf Wasserbasis verwendet, z.B. Suppen (insbesondere legierte Suppen), Bier, Eierlikör und sonstige übliche Getränke. Geeignet sind ferner Träger auf Milchbasis, wie Milch, Milchersatz, Joghurt, Kefir u.dgl.

Ein weiterer Vorteil besteht darin, dass **Edelfosin auch bei chemotherapieresistenten Tumoren** eingesetzt werden kann, da es einen völlig anderen Wirkungsmechanismus hat, der im Gegensatz zu den DNA-interaktiven Zytostatika primär an der Tumorzellmembran angreift und in die *signalling chain* der Zellen eingreift. Dadurch werden wichtige Enzyme, z.B. Phospholipase C, Proteinkinase C gehemmt, wodurch sich die Krebszelle nicht mehr teilen kann. Ferner bewirkt Edelfosin die **Re-Induktion der Apoptose** (vgl. Mollinedo & al. 1993); solchermaßen beeinflusste Zellen verlieren ihren "Unsterblichkeitsstatus" als Krebszelle.

### Pharmakodynamische Wirkung und therapeutische Wirksamkeit in vivo am Menschen

In Phase I-Studien wird nach Abschluss des tierexperimentelles Teils der Arzneimittelentwicklung die Verträglichkeit, Pharmakokinetik und Pharmakodynamik eines Medikamentes am Menschen geprüft und untersucht (Prüfung von Resorption, Verteilung im Körper, Abbauprodukte, Ausscheidung). Dadurch erhaltene Ergebnisse lassen jedoch keinen Schluss auf die therapeutische Wirksamkeit eines Medikamentes zu. Diese Aussage kann erst nach der Durchführung von Phase II-Studien gemacht werden.

Im Rahmen einer Phase II-Studie wurden die nachfolgend in Tabelle 1, mit ihrem Status vor und nach Behandlung aufgeführten Patienten mit Edelfosin behandelt. Wichtig ist dabei hervorzuheben, dass praktisch alle Patienten vorbehandelt waren oder von vorneherein nicht behandelbar waren (inoperable Tumoren). Alle Patienten wurden im Durchschnitt mit 300 mg Edelfosin/Tag (Schwankungsbreite zwischen 50 - 600 mg) behandelt. Dazu wurde das Lyophilisat in Wasser aufgelöst und in ein Trägermedium gegeben, das mindestens 3 - 3,5 % Fettgehalt aufwies und/oder einen vergleichbaren Anteil an Proteinen. Das Trägermdium-Wirkstoff-Gemisch wird von Patient zu Patient verschieden in unterschlichen Dosishöhen/Portion über den Tag verteilt getrunken. Die Applikation ist demnach oral; die Behandlung erfolgt gänzlich ambulant, sofern nicht das Krankheitsbild eine stationäre Aufnahme unumgänglich macht.

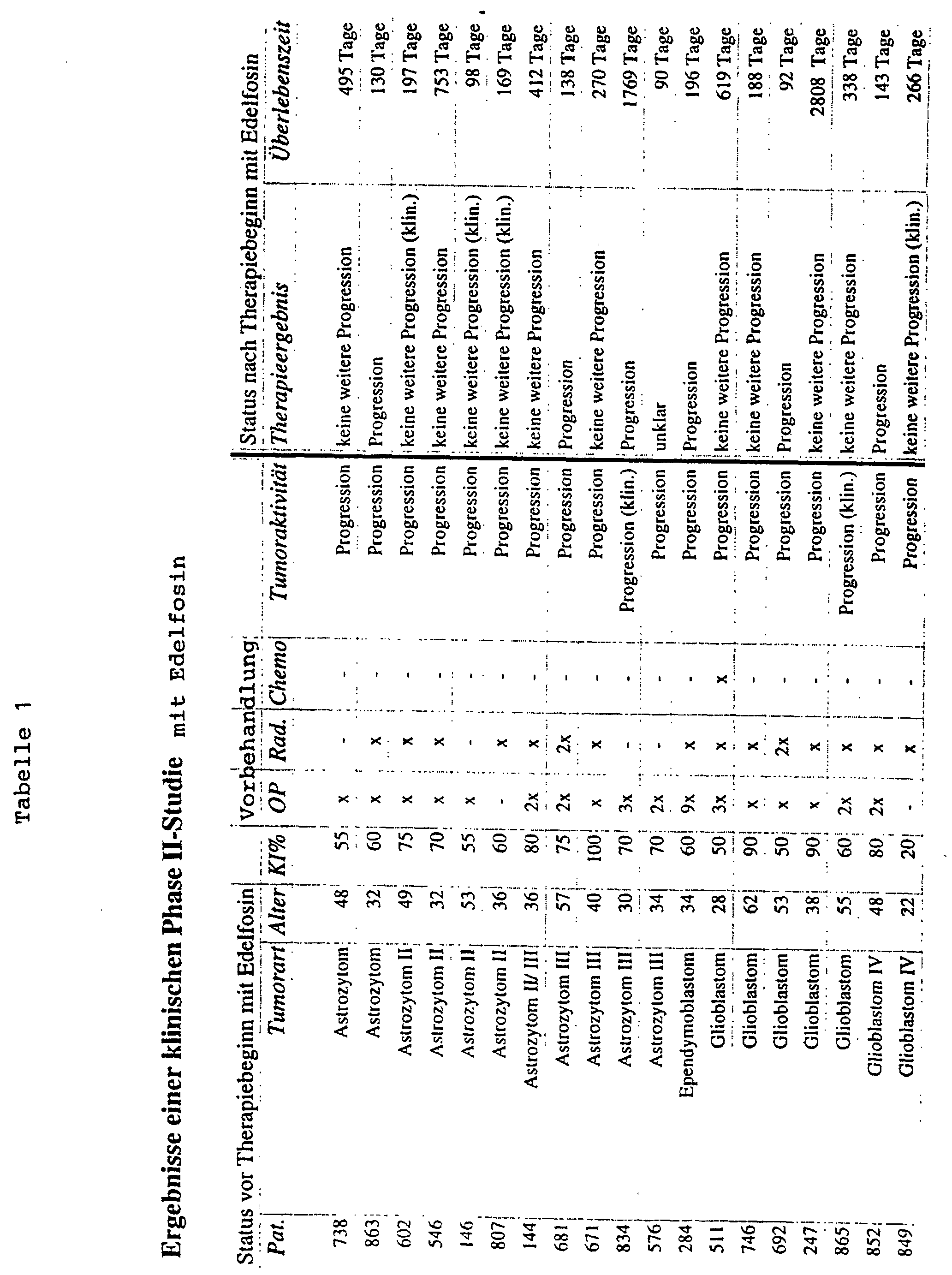

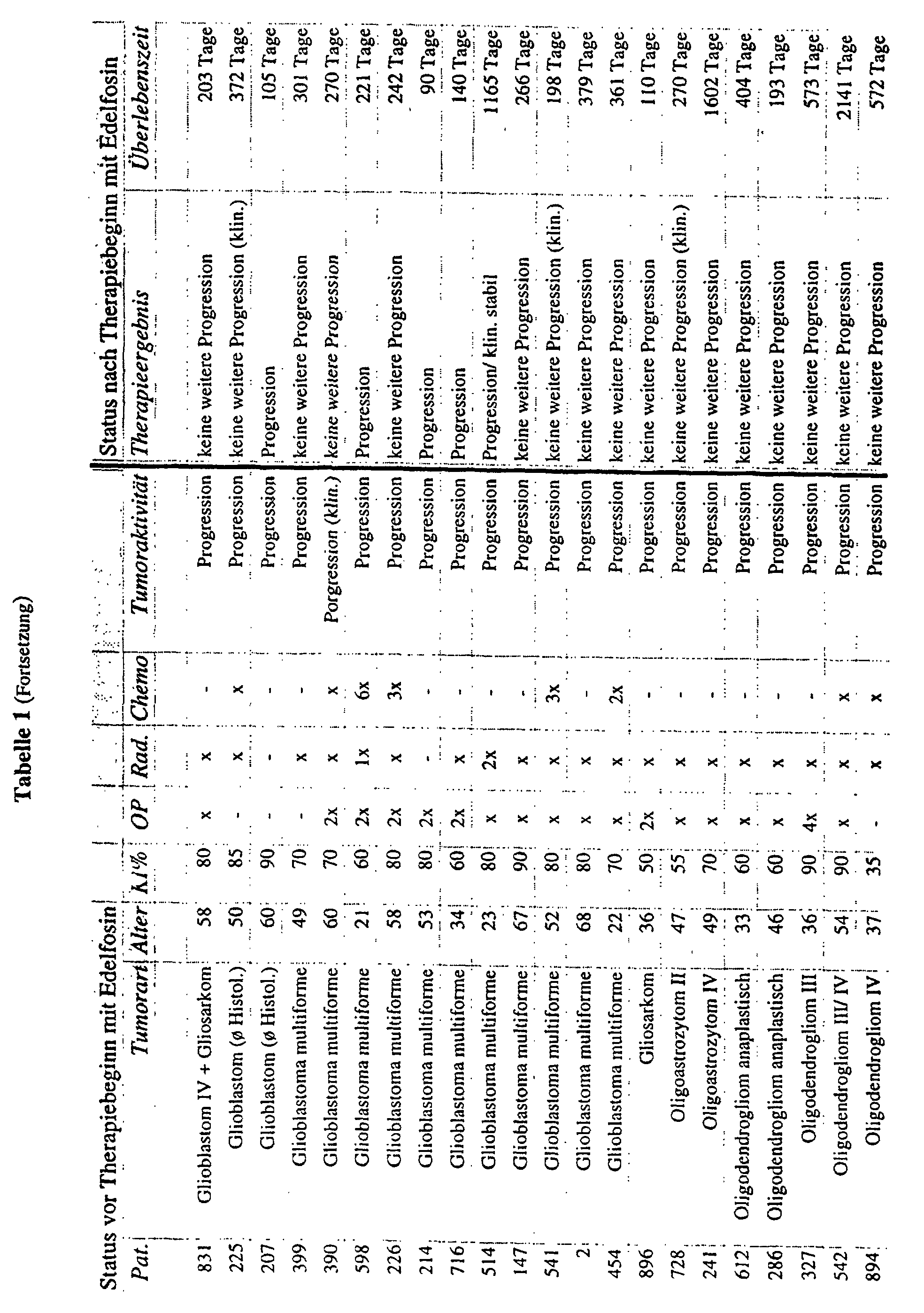

### Nachfolgend werden die Angaben dieser Tabelle näher erläutert:

| **Verteilung nach Tumorart** | |
|---|---|
| Astrozytome | N = 11 (26.8 %) |
| Glioblastome incl. Gliosarkome | N = 20 (48.8 %) |
| Oligoastrozytomel -dendrogliome | N = 7 (17.1 %) |
| andere Tumoren/ unklare Histologie (aber hirneigene Tumoren) | N = 3 (7.3 %) |
| | N =41 (100 %) |
| (vgl. Tabelle 1, Spalte "Tumorart") | |

### Alter

Die behandelten Patienten (N = 23 männlich, N = 18 weiblich) wiesen ein durchschnittliches Alter von 43.9 Jahren auf mit einem Minimum von 21 Jahren und einem Maximum von 69 Jahren. Dabei waren N = 18 Patienten (rd. 44 %) jünger als 40 Jahre, N = 18 (rd. 44 %) zwischen 40 und 59 Jahre alt und nur N = 5 Patienten (rd. 12 %) älter als 60 Jahre (vgl. Tabelle 1, Spalte "Alter ").

### Karnofsky-Index/ Allgemeinzustand (Kl %)

Bei Patienten mit Hirntumoren ist der Karnofsky-Index als Maß für den klinischen Zustand oder Allgemeinzustand ein wichtiger prognostischer Faktor, der bereits über Behandlung oder Nicht-Behandlung mit Zytostatika entscheidet. Generell wird empfohlen, eine adjuvante (!) Chemotherapie erst ab einem Karnofsky-Index von ≥ 70 % anzuwenden (vgl. Bogdahn & al. 1995). Der Allgemeinzustand wird üblicherweise nach der international verwendeten Karnofsky-Skala gemessen.

Der Karnofsky-Index lag im Durchschnitt bei den behandelten Edelfosin-Patienten bei 69.6 %, schwankte allerdings mit einer Streubreite von 20 - 100 % sehr stark. Immerhin wiesen N = 16 Patienten (rd. 39 %) einen Karnofsky-Index von < 70 % auf, N = 5 Patienten (rd. 12 %) gar einen Karnofsky-Index von ≤ 50 % (vgl. Tabelle 1, Spalte "KI%").
Hätte man sich an die allgemeine Regel gehalten, nur bei einem Karnofsky-Index ≥ 70 zu behandeln, so wären fast 39 % dieser Patienten mit einer durchschnittlichen Überlebenszeit von rd. 260 Tagen (vgl. Tabelle 1, Spalte "Überlebenszeit"), das sind immerhin mehr als 8 Monate, überhaupt nicht behandelt worden. Die Streubreite betrug dabei 90 - 619 Tage, wobei der maximale Überlebenszeitgewinn entsprechend bei > 20 Monaten lag.

### Vorbehandlung

Anzahl und Dauer von Vorbehandlungen vor Therapiebeginn mit einem Medikament sind entscheidend für die Bewertung der therapeutischen Wirksamkeit = Überlebenszeit und Überlebensqualität; die Überlebenszeit wird dabei stets von dem Zeitpunkt an gemessen, an dem die Therapie mit einem Medikament begonnen wird. Wieviel Zeit ab Diagnose bis zum Behandlungsbeginn verstrichen ist, spielt dabei zunächst keine unmittelbare Rolle. Sie ist aber dann zu beachten, wenn die Überlebenszeit einer ultima ratio-Therapie (wie hier Edelfosin) mit derjenigen von Therapien verglichen wird, die in aller Regel unmittelbar nach Diagnose begonnen werden (das ist der Fall für Chemotherapie- und Radiatio-Studien).

Das mit Edelfosin behandelte Patientenkollektiv war in rd. 85 % mehrfach vorbehandelt; d.h. es war mehr als eine Behandlungsart von den Möglichkeiten einer Operation, Bestrahlung oder Chemotherapie vor Beginn der Therapie mit Edelfosin bereits angewendet worden. Alternative Therapieverfahren (Mistel, Hyperthermie etc.) wurden nicht gewertet.

Aufgeschlüsselt nach Operation, Bestrahlung und Chemotherapie bedeutet dies, dass bei rd. 85 % der Patienten ein Rezidiv oder Resttumor nach erfolgloser Operation (auch mehrfacher) zur Aufnahme der Behandlung mit Edelfosin führte. Eine Bestrahlung (auch mehrfach) lag ebenfalls in rd. 85 % aller Patienten vor; eine Chemotherapie war bei rd. 22 % der Patienten angewandt worden, bevor die Therapie mit Edelfosin begonnen wurde.

Bei praktisch allen Patienten führte ein nach allen angewandten Therapien weiterwachsender Tumor zur Therapie mit Edelfosin, d.h. der Tumor war progredient (vgl. Tabelle 1, Spalte "Tumoraktivität"). Die Präzisierung "klinische" Progression bedeutet hierbei, dass der Tumor aufgrund einer sich verschlechternden klinischen Symptomatik (z.B. Zunahme von Kopfschmerzen, Kopfdruck, Schwindel, Fallneigung, neurologische Symptomatik allgem.) progredient war, eine vergleichende objektive Aufnahme (z.B. CT) aber nicht vorlag.

### Pharmakodynamische Wirkung (Therapieergebnis)

Die pharmakodynamische Wirkung, d.h. die Wirkung eines Medikamentes direkt am Tumor, wird üblicherweise 2 Monate nach Beginn einer Therapie gemessen. Je nach der Substanzklassen-Zugehörigkeit (also Zytostatikum, Hormonpräparat, Immunomodulator etc.) wird diese pharmakodynamische Wirkung unterschiedlich festgelegt. Edelfosin ist ein *phenotype modifier*/*biological response modifier* und führt somit nicht direkt zur unmittelbaren Abtötung einer Zelle, vielmehr zu einer Hemmung ihrer Teilung, zur Re-Induktion der Apoptose und/ oder zur Ausdifferenzierung in eine "normale" Zelle. Das Therapieziel ist entsprechend ein Tumorwachstumsstillstand (no change).

Bei den behandelten Patienten mit einem Hirntumor zeigte sich in einem hohen Prozentsatz eine Stabilisierung des Tumorgeschehens (siehe Tabelle 1, Spalte "Therapieergebnis").

Objektiv bewertet durch eine bildgebende Aufnahme nach 2 Monaten Therapie zeigte sich bei rd. 56 % der behandelten Patienten ein Tumorwachstumsstillstand (NC = no change). Bei weiteren rd. 14 % wurde zwar keine objektive Aufnahme gemacht, zeigte sich aber aufgrund der klinischen Symptomatik eine Stabilisierung (cNC = clinical no change). Dies ist in einer Palliativsituation, in der eine Heilung nicht mehr möglich ist, oftmals das einzige anzustrebende Therapieziel; auf eine rein palliative Behandlung muss sich aber ein Therapeut bei inoperablen, rezidivierten oder metastasierten Tumoren ohnehin beschränken. Wird diese Linderung (Palliation) dazu noch unter weitgehend fehlenden unerwünschten Arzneimittelwirkungen ( = Nebenwirkungen) erreicht, dann entspricht auch die klinische Stabilisierung einem echten therapeutischen Gewinn.

In rd. 24 % aller behandelten Patienten war der Tumor auch nach 2 Monaten noch progredient, wobei bei 2% insofern ein diskrepanter Befund vorlag, als sich klinisch eine Stabilisierung/Besserung eingestellt hatte, gleichwohl der Tumor nach bildgebenden Verfahren progredient war.

Im Rahmen von Phasell-Studien wurden lege artis auch die unerwünschten Arzneimittelwirkungen (UAW) untersucht. Das Ergebnis zeigt Tabelle 2:

**Tabelle 2**

| Gradeinteilung | 0 | Grad 1 | | | Grad 2 | | | Grad 3 | | | Grad 4 | | | φ Bewert | Verläufe |
|---|---|---|---|---|---|---|---|---|---|---|---|---|---|---|---|
| **Symptom** | | ja | ? | nein | ja | ? | nein | ja | ? | nein | ja | ? | nein | | |
| | | | | | | | | | | | | | | | |
| **Appetit** | 123 | 4 | 5 | 4 | 1 | 2 | 1 | 2 | 2 | 4 | - | - | 1 | 1 | 150 |
| **Übelk./Erbrech.** | 121 | 12 | 5 | 1 | 2 | 6 | 8 | - | - | 2 | - | - | - | 2 | 159 |
| **Diarrhoe** | 142 | 5 | 1 | - | 1 | - | 1 | - | - | - | - | - | - | - | 150 |
| **Obstipation** | 143 | 3 | 3 | 1 | - | - | - | - | - | - | - | - | - | - | 150 |
| **Stomatitis** | 149 | - | - | - | - | - | - | - | - | - | - | - | - | - | 149 |
| **Fieber** | 142 | - | - | 1 | - | - | 7 | - | - | - | - | - | - | - | 150 |
| **Infektion** | 146 | - | - | 3 | - | - | 1 | - | - | - | - | - | - | - | 150 |
| **Dyspnoe** | 141 | - | - | 4 | - | - | 3 | - | - | 1 | - | - | 1 | - | 150 |
| **Allergie** | 151 | - | - | - | - | - | - | - | - | - | - | - | - | - | 151 |
| **Hautveränder.** | 145 | - | - | - | - | - | - | - | - | - | - | - | 3 | - | 148 |
| **Alopezie** | 125 | - | 1 | 3 | - | - | 1 | - | - | 3 | - | - | 16 | - | 149 |
| **ZNS-Toxizität** | 133 | - | 1 | 1 | - | - | - | - | - | - | - | - | - | - | 135 |
| **Periph. Neurop.** | 131 | - | 1 | 1 | - | - | - | - | - | - | - | - | - | - | 149 |
| **Herzinsuffizienz** | 150 | - | - | - | - | - | - | - | - | - | - | - | - | - | 150 |
| **Rhythmusstör.** | 145 | - | - | 5 | - | - | - | - | - | - | - | - | - | - | 150 |
| **Hämaturie** | 149 | - | - | - | - | - | - | - | - | - | - | - | - | - | 149 |
| **Blutungen** | 148 | - | - | 2 | - | - | - | - | - | - | - | - | - | - | 150 |
| **Schmerzen** | 133 | - | - | 7 | - | - | - | 5 | - | 3 | - | - | - | 1 | 149 |

Auflistung der unter Therapie mit Edelfosin berichteten unerwünschten Arzneinüttelwirkungen (UAW)
Grad 0 = keine Beschwerdemeldung
Grad ../ ja = durch Edelfosin verursachte UAW
Grad ../ ? = fraglich durch Edelfosin verursachte UAW
Grad ../ nein = nicht durch Edelfosin verursachte UAW
φ Bewert. = es wurde keine Bewertung eingetragen

Zur Bewertung der Schweregrad = Gradeinteilungen 1 - 4 (leicht bis schwer)

### Erläuterung:

Die Gradeinteilung der Symptome, wie sie auf die Prüfbögen übertragen wurden, erfolgte nach der international verwendeten Einteilung der WHO.

Die Bewertung "ja" bedeutet, dass ein Symptom durch das Medikament verursacht wird. Die Bewertung "?" (fraglich) bedeutet, dass die Ursache für die Beschwerdemeldung unklar ist; die Bewertung "nein" bedeutet, dass das Symptom eindeutig durch die Krankheit und nicht durch das Medikament bedingt ist.

Oftmals sind bestimmte Krankheitsbilder mit ganz bestimmten Symptomen verbunden, so dass eine eindeutige Bewertung der Ursache für ein Symptom nicht immer möglich ist. "Übelkeit/ Erbrechen" sind gerade bei primären Hirntumoren je nach Lokalisation des Tumors oft tumor-induziert und nicht medikamenten-induziert.

Typisch für die durch die Therapie mit Edelfosin verursachten UAWs ist ihre kurze Dauer. UAWs treten oftmals in den ersten beiden Therapiemonaten auf und klingen dann gänzlich wieder ab. In jedem Fall aber sind die beobachteten Symptome innerhalb kürzester Zeit (Stunden) reversibel. Besonders hervorzuheben aber ist, dass alle gemeldeten UAWs stets ohne pathologisches Korrelat sind. So wiesen z.B. entsprechende Untersuchungen der Speiseröhre und des Magens (Ösophagoskopie, Gastroskopie) bei Patienten, die unter Therapie mit Edelfosin unter Übelkeit/Erbrechen ("Magendruck") gelitten hatten, keinerlei pathologische Befunde auf. Viele Patienten erfahren durch die Therapie überhaupt keine UAWs. Pat. Nr. 542 litt während der fast 6 Jahre dauernden Einnahme von Edelfosin unter keinerlei UAWs, während Pat. Nr. 247 unter Therapie über Übelkeit/ Appetitmangel" klagte, dies allerdings noch heute, d.h. viele Jahre nach Beendigung der Therapie (überlebt mittlerweile im 8. Jahr) tut.

Eine kumulative Toxizität tritt definitiv auch nach mehreren Jahren ununterbrochener Therapie nicht auf und ist aufgrund des selektiven Wirkmechanismus auch nicht zu erwarten.

Damit ist im Gegensatz zu einer nur stationär durchführbaren Behandlung bekannter Art durch die erfindungsgemäße Therapie mit Edelfosin ein Höchstmaß an Lebensqualität gewährleistet.

### Therapeutische Wirksamkeit

Die therapeutische Wirksamkeit wird vor allem in Form von Überlebensqualität und Überlebenszeit gemessen. Es ist heute unumstritten, dass die Messung einer pharmakodynamischen Wirkung allein (komplette Remission/ partilelle Remission/ no change) zwar innerhalb von Therapiestudien wichtig ist, aber für den Patienten per se keinen Wert zu haben braucht. Dies trifft ganz besonders für die Palliativsituation zu, in der eine Heilung nicht mehr möglich ist. Eine mögliche Therapiewirkung muss dann besonders sorgfältig in Relation zu den Nebenwirkungen einer Behandlung gesetzt werden, denn bei der palliativen Therapie haben die Nebenwirkungen eines Medikamentes ein ungleich größeres Gewicht als bei der kurativen Therapie. Ziel der Palliation ist fraglos auch das Anstreben einer Verlängerung der Überlebenszeit und/ oder die Verbesserung der Lebensqualität, idealerweise die Kombination von beidem.

Die Überlebenszeit wird ab Therapiebeginn mit einem Medikament gemessen. Die Daten der *state of the art*-Überlebenszeiten, wie sie für die Behandlung von Hirntumoren in der Literatur angegeben werden, basieren in aller Regel auf der Behandlung von Patienten entweder adjuvant, also unmittelbar nach operativer Entfernung eines Tumors, oder aber auf einer Behandlung direkt nach Diagnosestellung.

Die mit Edelfosin behandelten Patienten wiesen alle eine mehr oder weniger lange Anamnese auf, d.h. sie waren größtenteils mehrfach vorbehandelt.

Bereits insofern ist ein Vergleich der in Tabelle 1 angegebenen Überlebensdaten (vgl. Tabelle 1, Spalte "Überlebenszeit") mit Literaturdaten sehr schwierig oder schlicht nicht möglich. Die in Tabelle 1 präsentierten Daten entsprechen vielmehr einer *ultima ratio*-Therapiesituation mit einer entsprechend durch die Vorbehandlung bereits verkürzten Überlebenszeit.

Ein Vergleich ist auch deswegen schwierig, weil für die vorliegende Studie nicht das üblicherweise hochselektierte Patientengut, wie es regelmäßig für klinische Studien rekrutiert wird, herangezogen wurde. Behandelt wurde vielmehr jeder Patient, bei dem schulmedizinische Behandlungsmöglichkeiten ausgeschöpft waren, aber weiterhin Therapiebedarf vorlag.

Die durchgeführten Untersuchungen ergaben, daß es keine Rolle spielt, ob die im Hirn lokalisierten Metastasen von soliden Tumoren (z.B. Lungenkarzinome, Mammakarzinome, colorektale Karzinome etc.) stammen oder von nicht-soliden Tumoren (z.B. Lymphome, Leukämien etc.). Es sei nur am Rande erwähnt, dass Patienten mit Hirnmetastasen, z.B. ausgehend von einem nicht-kleinzelligen Bronchialkarzinom, in der Regel noch 6 Monate Überlebenszeit haben und Hirnmetastasen üblicherweise als Ausschlusskriterium für Chemotherapie-Studien gelten.

### Quellen

*Bauchinger, M. & al. (1983).* Cytogenetic effects of an alkyllysophospholipid derivative in human peripheral lymphocytes exposed in vitro and in vivo. Mutation Research 121: 225 ff.

*Berdel, W.E. & al. (1983).* Experimental chemotherapy of radiation injury with synthetic lysophospholipid analogs in mice. Radiation Research 94: 166 ff. *Berdel, W.E. & al. (1984).* Cytotoxic effects of alkyl-lysophospholipids in human brain tumor cells. Oncology 41: 140 ff.

*Berger, M.R. & al. (1984).* Influence of the alkyl-lysophospholipid ET 18 OCH3 on methylnitrosourea-induced rat mammary carcinomas. Oncology 41: 109 ff.

*Berens, M. & al. 1993.* Effects of structural modifications of ether lipids on antiproliferative activity against human glioma cell lines. Anticancer Research 13: 401 ff.

*Bogdahn, U. & al. (1995).* Kapitel V-2.19 Maligne Gliome. In: Zeller, W.J. & H. zur Hausen (Hrsg.). Onkologie. Ecomed Verlag, Landsberg.

*Carmustin.* Unerwünschte Arzneimittelwirkungen gemäß aktueller Fachinformation. *De Vita, V.T. & al. (1997).* Cancer - Principles & Practice of Oncology. Chapt. 42, Neoplasms of the Central Nervous System. 5th ed., Lippincott-Raven, Philadelphia, New York.

*Engebraaten, O. & al. (1991).* Effect of alkyllysophospholipid on glioblastoma cell invasion into fetal rat brain tissue in vitro. Cancer Research 51: 1713 ff.

*Hickman, J.A. (1992).* Membrane and Signal Transduction Targets. In: Workman, P. (Ed.), New Approaches in Cancer Pharmakology: Drug Design and Development. Springer Verlag, Berlin.

*King, M.* & *al. (1981).* Failure to detect mutagenic effects of anti-tumor alkyl-lysophospholipids. Canc. Lett. 12: 217 ff.

*Magistrelli, A. & al. (1994).* Fate of 1-*O*-octadecyl-2-*O*-methyl-*rac*-glycero-3-phosphocholine (ET18-OMe) in malignant cells, normal cells, and isolated and perfused rat liver. Drug Metabolism and Disposition 23(1): 113 ff.

*Mollinedo*, *F. & al. (1993).* Early and selective induction of apoptosis in human leukemic cells by the alkyl-lysophospholipid ET-18-OCH3. Biochemical and Biophysical Research Communications 192 (2): 603 ff.

*Snyder, F. & al. (1991).* Membrane-targeted biochemical effects and the role of cellular differentiation in the selective antitumor actions of alkylmethoxyglycerophosphocholine. In: Honn, K.V. (eds). Eicosanoids and other bioactive lipids in cancer and radiation injury. Kluwer Acad. Publ., Boston/ Dodrecht/London.

*WHO (1994).* Essential drugs for cancer chemotherapy. Bulletin of the World Health Organization 72(5): 693 ff.

## Patentansprüche

1. Verwendungvon 1-Octadecyl-2-methyl-sn-glycero-3-phosphocholin zur Herstellung eines Arzneimittels zur oralen Verabreichung zur Behandlung von Hirntumoren.

2. Verwendung des Wirkstoffs gemäß Anspruch 1 in einem flüssigen trinkbaren Trägermaterial aufgelöst.

3. Verwendung nach Anspruch 2 in einem flüssigen Trägermaterial das mindestens 3 Gew.-% Fett oder/und Protein enthält.

4. Verwendung nach Anspruch 2 oder 3, **dadurch gekennzeichnet, dass** das Trägermaterial ein Wasserbasisgetränk, insbesondere Milch ist.

5. Verwendung nach Anspruch 1 in einer Wirkstoffmenge entsprechend einer Tagesdosis von 50 bis 600 mg pro Person.

## Claims

1. Use of 1-octadecyl-2-methyl-sn-glycero-3-phosphocholine for the preparation of a medicament for oral administration in the treatment of brain tumours.

2. Use of the active ingredient according to Claim 1 dissolved in a liquid drinkable carrier material.

3. Use according to Claim 2 in a liquid carrier material which contains at least 3 % by weight of fat and/or protein.

4. Use according to Claim 2 or 3, **characterised in that** the carrier material is a water-based beverage, in particular milk.

5. Use according to Claim 1 in a quantity of active ingredient corresponding to a daily dose of 50 to 600 mg per person.

## Revendications

1. Utilisation de 1-octadécyl-2-méthyl-n-glycéro-3-phosphocholine pour la fabrication d'un médicament à administration orale destiné au traitement de tumeurs du cerveau.

2. Utilisation du principe actif selon la revendication 1 dissous dans une substance porteuse liquide buvable.

3. Utilisation selon la revendication 2 dans une substance porteuse liquide qui contient au moins 3 % en masse de lipides et/ou de protéines.

4. Utilisation selon la revendication 2 ou 3 **caractérisée en ce que** la substance porteuse est une boisson à base aqueuse, en particulier le lait.

5. Utilisation selon la revendication 1 avec une quantité de principe actif correspondant à une dose journalière de 50 à 600 mg par personne.
